(19) Europäisches Patentamt
European Patent Office
Office européen des brevets

(11) Publication number: **0 216 499 A2**

(12) **EUROPEAN PATENT APPLICATION**

(21) Application number: 86306416.8

(22) Date of filing: 19.08.86

(51) Int. Cl.[4]: C 08 F 8/32
C 08 F 20/54, C 12 N 11/08

(30) Priority: 23.08.85 GB 8521192

(43) Date of publication of application: 01.04.87 Bulletin 87/14

(84) Designated Contracting States: AT CH DE FR GB LI NL

(71) Applicant: COURTAULDS PLC
18, Hanover Square
London W1A 2BB(GB)

(72) Inventor: Radley, Peter Michael
9 Whitemoors Road Stoke Golding
Nuneaton Warwickshire(GB)

(74) Representative: Hale, Stephen Geoffrey et al,
J.Y. & G.W. Johnson Furnival House 14/18 High Holborn
London WC1V 6DE(GB)

(54) **Polymeric carrier materials.**

(57) Water-insoluble porous polymer beads suitable for use as carrier materials for the immobilization of enzymes and other biologically active materials contain functional groups bonded to the polymer as pendent groups of the formula:

```
 O R
 ‖ |
-C-N-A-X
```

where A represents a divalent aliphatic linking group having 2 to 10 carbon atoms, X represents a reactive functional group and R represents a hydrogen atom, an alkyl group or a group of the formula -A-X. They are prepared by a process in which an unsaturated nitrile monomer and a cross-linking monomer containing at least two polymerisable double bonds are copolymerised in an organic phase dispersed in water, in which the organic phase comprises an inert, substantially water-immiscible organic liquid in which the monomers are soluble, to form water-insoluble porous polymer beads, and the polymer beads so formed are reacted with an amine of the formula:

```
  R
  |
H-N-A-X
```

where A, X and R are defined as above in the presence in water, to convert at least some of the nitrile groups of the polymer to groups of the formula:

```
 O R
 ‖ |
-C-N-A-X
```

and the beads are then optionally reacted with a reagent, preferably an aldehyde, capable of bonding to a biologically active material.

EP 0 216 499 A2

# Polymeric carrier materials

This invention relates to polymeric carrier materials useful in the immobilisation of enzymes and other biologically active materials and to the preparation of such carrier materials. The biologically active materials can be bonded to the polymeric carrier, which is in the form of water-insoluble porous beads, by covalent bonds. If the biologically active materials are then used in a chemical or biochemical process they can as a result readily be separated from the product of that process and retained for further use.

EP-A-129,719 describes a polymer useful in enzyme immobilisation which is derived from an N-aminoalkyl or N-hydroxyalkyl amide monomer of the formula:-

$$CH_2=C(X)-CO-NH-R-Y$$

in which X is hydrogen or methyl, R is an aliphatic hydrocarbon radical having 1 to 12 carbon atoms and Y is a hydroxyl or amine group, and at least one other copolymerisable monomer. The N-aminoalkyl or N-hydroxyalkyl amide monomers are generally hydrophilic and miscible with water and polar organic solvents. They are generally copolymerised with another monomer which is hydrophilic as well as with a cross-linking monomer. The polymerisation is carried out in dispersion in an organic liquid, preferably a paraffin having 6 to 20 carbon atoms. The monomers are mixed with an inert polar liquid which is insoluble in the dispersion medium, for example dimethyl formamide, dimethyl sulphoxide, dioxan or water; this helps to develop a porous bead structure.

The N-aminoalkyl amide or N-hydroxyalkyl amide functional groups of the polymers of EP-A-129,719 react readily with spacer compounds which introduce epoxide or other reactive groups which can be bonded to the biologically active material. The polymer beads of EP-A-129,719 are, however, limited in their application in that they are necessarily formed from hydrophilic monomers; hydrophobic monomers would

dissolve in the dispersion medium and not become incorporated in the polymer beads. There is a need for a process capable of forming porous beads of polymeric carrier material which are more hydrophobic than those produced in EP-A-129,719, and in which the hydrophobic/hydrophilic balance of the polymer can be readily varied.

A process according to the invention for the preparation of water-insoluble porous polymer beads useful in the immobilisation of biologically active materials, the polymer containing functional groups bonded to the polymer as pendent groups of the formula:

$$-\overset{O}{\overset{\|}{C}}-\overset{R}{\overset{|}{N}}-A-X$$

where A represents a divalent aliphatic linking group having 2 to 10 carbon atoms, X represents a reactive functional group and R represents a hydrogen atom, an alkyl group (e.g. of 1 to 6 carbon atoms) or a group of the formula -A-X, is characterised in that an unsaturated nitrile monomer and a cross-linking monomer containing at least two polymerisable double bonds are copolymerised in an organic phase dispersed in water, in which the organic phase comprises an inert, substantially water-immiscible organic liquid in which the monomers are soluble, to form water-insoluble porous polymer beads, and the polymer beads so formed are reacted with an amine of the formula:

$$H-\overset{R}{\overset{|}{N}}-A-X$$

where A, X and R are defined as above, in the presence of water, to convert at least some of the nitrile groups of the polymer to groups of the formula:

$$-\overset{O}{\overset{\|}{C}}-\overset{R}{\overset{|}{N}}-A-X \, .$$

The unsaturated nitrile monomer is preferably acrylonitrile or methacrylonitrile. It is preferred that the

amount of unsaturated nitrile monomer present in the reaction mixture is at least 50 mole per cent, more preferably at least 70 mole per cent, based on the total amount of monomers present, so that units of the nitrile monomer preferably form at least 50 mole per cent, more preferably at least 70 per cent, of the copolymer.

The cross-linking monomer is preferably a hydrophobic water-insoluble monomer such as divinyl benzene, although monomers which are more hydrophilic such as N,N-methylene bis(acrylamide), N,N-ethylene bis(acrylamide) or N,N-hexamethylene bis(acrylamide), can also be used.

The preferred amount of cross-linking monomer and thus the preferred amount of cross-linking in the polymer formed depends on the application of the polymer. In general increasing mechanical strength is conferred by increasing cross-linking. A highly cross-linked polymer is preferred for reactions in a column, where a non-deformable bead is required. A more lightly cross-linked polymer may be more suitable for a reaction in which the polymer beads are stirred in the reaction medium. The cross-linking monomer preferably forms from 2 to 40 mole per cent of the monomer units, most preferably 5 to 10 mole per cent. The amount of cross-linking monomer determines the maximum amount of reactive groups which can be incorporated in the polymer. Increasing amounts of cross-linking monomer may also reduce the pore size of the polymer beads produced.

The inert, water-immiscible organic liquid present in the polymerisation reaction is preferably a hydrocarbon, for example an aromatic hydrocarbon such as toluene, benzene or xylene or a paraffinic hydrocarbon such as hexane or heptane. The amount of inert organic liquid is preferably 5 to 300 per cent by volume based on the monomers, most preferably 40 to 100 per cent. Increasing amounts of inert

organic liquid increase the porosity and pore size of the polymer beads produced.

The polymerisation reaction is generally carried out in the presence of a free radical polymerisation initiator which is soluble in the dispersed organic phase and which is preferably insoluble in water, for example an organic peroxide such as benzoyl peroxide or an azo compound such as azobisisobutyronitrile. The temperature of polymerisation is preferably in the range 50 to 120°C, more preferably 60 to 90°C, and the polymerisation time is preferably in the range 1 to 24 hours.

The monomers and inert, water-immiscible liquid are preferably dispersed in water with the aid of a dispersion stabiliser such as gelatin, a long chain alkyl sulphate salt such as sodium lauryl sulphate, or a block copolymer. Stirring of the dispersion is generally maintained during the polymerisation reaction. The rate of stirring influences the size of the polymer beads produced, faster stirring leading to smaller polymer beads. If the rate of stirring is kept constant, beads having a narrow particle size range can be produced. The average particle size of the polymer beads is preferably in the range 50 to 800 microns. At least 50 per cent of the beads, preferably at least 70 per cent, should preferably have a diameter which is within 25 per cent of the average.

The nitrile monomer and the cross-linking monomer can be copolymerised with at least one further monomer which is partially soluble and preferably substantially soluble in the inert, water-immiscible organic

liquid and substantially insoluble in water. The monomer may be chosen to increase the hydrophobic nature of the polymer beads. Examples of hydrophobic monomers includes styrene, alpha-methyl styrene, ethyl styrene, vinyl pyridine, alkyl acrylate and methacrylate esters having more than 3 carbon atoms in the alkyl group and vinyl esters of a carboxylic acid having more than 4 carbon atoms. Such further monomers if present may form up to 50 mole per cent of the total monomers, preferably 5 to 30 per cent.

The polymer is produced in the form of a suspension of substantially spherical polymer beads in water, which can be removed by filtration. The beads are preferably washed with water and with an inert organic solvent, which may be the same as the water-immiscible liquid present during polymerisation, and are subsequently dried. At least part of the water-immiscible organic liquid becomes incorporated in the polymer beads during polymerisation but is removed during working up of the product, to leave pores in the polymer beads. The pore size of the beads is generally in the range 0.01 to 1 micron as determined for example by electron microscopy or mercury porosimetry.

The porous beads of cross-linked nitrile copolymer produced are then reacted with an amine of the formula:

$$H-\underset{}{\overset{\displaystyle R \atop |}{N}}-A-X$$

in the presence of water to convert the nitrile groups to functional groups of the formula:

$$-\overset{\displaystyle O \atop \|}{C}-\overset{\displaystyle R \atop |}{N}-A-X$$

A, R and X being defined as above. The amine is preferably a primary amine of the formula:

$$H_2N-A-X$$

which reacts to form functional groups of the formula:

$$-\overset{O}{\overset{\|}{C}}-\overset{H}{\overset{|}{N}}-A-X$$

The divalent linking group A can be an alkylene group or can contain one or more ether (O) or amine (NH or N-alkyl) linkages. Preferred examples of reactive functional groups X are amine or hydroxyl groups. The amine is preferably ethylene diamine, ethanolamine, 1,2- or 1,3- propylene diamine, hexamethylene diamine, diethylene triamine (which reacts mainly through one of its primary amine groups) or propanolamine. Diethanolamine is an example of a secondary amine which reacts to introduce two functional groups of the type -A-X. Ethylethanolamine is an example of a secondary amine introducing an alkyl group R.

The reaction between the beads and the amine can be carried out in the presence of an excess of amine and of water, for example by stirring the polymer beads in a mixture of amine and water. The reactants are preferably heated at a temperature of 80 to 150°C and the time of reaction can be for example 3 to 24 hours or more. The reaction is sufficiently slow that the degree of conversion of nitrile groups to substituted amide groups can be controlled by varying the time and temperature of reaction, for example to achieve a desired conversion in the range 25 to 100 per cent. Alternatively the degree of conversion of nitrile groups can be limited by using less than a stoichiometric amount of the amine. The groups

$$-\overset{O}{\overset{\|}{C}}-\overset{R}{\overset{|}{N}}-A-X$$

increase the hydrophilic nature of the polymer beads whereas the nitrile groups remaining in the polymer reduce the hydrophilic nature of the polymer beads. The process of the present invention thus allows two ways of varying the hydrophobic/hydrophilic balance of the polymer beads without affecting the pore structure and degree of cross-linking of

the polymer. The hydrophobic/hydrophilic balance can be varied by the use of a hydrophobic comonomer such as styrene or by controlling the degree of conversion of nitrile groups to substituted amide groups. Thus in another aspect the present invention provides water-insoluble porous polymer beads comprising a cross-linked polymer having both nitrile pendent groups and substituted amide pendent groups of the formula:

```
 O R
 ‖ |
-C-N-A-X
```

where A, X and R are as defined above.

The efficiency of bonding of different enzymes to a polymeric carrier depends on the balance of hydrophobic and hydrophilic groups in the polymer, and different enzymes may require different hydrophobic/hydrophilic balances. For example, hydrophobic carriers have advantages in immobilising lipid enzymes. In a hydrophilic water-swellable polymer mechanical strength of the beads and resistance to swelling is conferred by cross-linking. In general, increasing cross-linking decreases water-swellability and increases the chemical strength. However, the degree of cross-linking also affects the pore size. An adequate mechanical strength may require a degree of cross-linking which leads to an inadequate pore size. The presence of hydrophobic monomer units, which may be derived from a hydrophobic comonomer such as styrene or from unconverted nitrile groups, in the polymer will decrease water-swellability and increase mechanical strength without affecting the cross-linking and the pore size. Moreover, for many purposes it is desired that the immobilised enzyme should be dried. Drying the highly hydrophilic polymer beads of EP-A-129,719 causes shrinkage which may damage the enzyme. This can be avoided with a more hydrophobic polymer.

The reactive functional groups X introduced into the polymer molecule may themselves be suitable for bonding to an enzyme or other biologically active material or may be further reacted with a reagent for introducing such groups. When the group X is amine or hydroxyl it is usually not suitable for reacting directly with an enzyme and the polymer beads are further reacted with a compound capable of introducing more reactive groups, for example aldehyde, epoxide, cyanate, isocyanate, carboxylic acid, acid chloride or ester or acetal groups so that the polymer beads have pendant groups containing a reactive group capable of bonding to a biologically active material. For example a polymer containing $-CO-NH-A-NH_2$ groups can be reacted with a dialdehyde such as glutaraldehyde, for example at a temperature of up to 60°C and a time of from 2 to several hours or longer, to introduce aldehyde groups. The polymer can then be bonded to the biologically active material through the aldehyde groups. Alternatively, a polymer containing amine groups can be reacted with an epihalohydrin, for example epichlorhydrin, or a diepoxide, for example at 50-200°C for 2-6 hours in the presence of a basic catalyst to introduce epoxide groups, or it can be reacted with a dialkyl diester to introduce alkyl ester groups. A polymer containing groups of the formula: -CO-NH-A-OH can be reacted with cyanogen bromide, for example at up to 50°C in water in the presence of a basic catalyst at pH 9-11, to form -CO-NH-A-OCN groups; the cyanate groups can bond to the biologically active material. Alternatively, a polymer containing hydroxyl groups can be reacted with epichlorhydrin to introduce epoxide groups.

The proportion of pendent groups in the polymers beads that contain a reactive group capable of bonding to a biologically active material depends upon the proportion of

nitrile groups converted to substituted amide groups, which, as mentioned above depends upon, for example, the desired hydrophobic/hydrophilic balance of the polymer beads. Thus, in a further aspect the present invention provides water-insoluble porous polymer beads having both nitrile pendent groups and pendent groups containing a reactive group capable of bonding to a biologically active material.

The polymeric carrier material thus treated is preferably reacted with the biologically active material by contacting the activated polymer beads with an aqueous solution of the biologically active material, typically for 30 minutes to a few hours. The temperature of reaction is determined by the sensitivity to temperature of the biologically active material. Temperatures of below 30°C are usual. After the reaction is substantially complete the polymer beads may be washed, usually with water or with a solution having an appropriate pH value, to remove any unbound material. Examples of biologically active materials thus can be bound to the polymeric carrier beads include enzymes, substances which bind to enzymes such as inhibitors, substrates, cofactors and proteins, peptides, antigens, certain dyes and nucleotides.

The invention is illustrated by the following examples:-

<u>Example 1</u>

1 (a) <u>Preparation of Nitrile-Containing Polymer Beads Using Acrylonitrile, Divinylbenzene and Ethylstyrene Monomers</u>

A mixture of distilled water (250g), calcium carbonate

(2.5g), sodium sulphate (15g), a 3 per cent gelatin solution (10mls), toluene (40mls) acrylonitrile (40.3g) and a 1:1 mixture of divinylbenzene and ethylstyrene (13.8g) was stirred at room temperature. A portion (0.6g) of benzoyl peroxide containing 25 per cent water was added.

The mixture was warmed to 62°C and held close to that temperature for 2.5 hours. The mixture was further heated to 71°C, held for 1 hour, heated to 82°C and held for 1 hour. After cooling, the solid polymer beads were filtered and washed successively with water and toluene. After drying, there was 47.2g solid product.

The product was shown by microscopy to be spherical with 71 per cent by number of spheres having a diamenter between 0.2mm and 0.3mm. Electron microscopy demonstrated the porosity of the beads.

1 (b) Reaction of Polymer with Ethylene Diamine

A sample of the polymer beads prepared above (10g) was suspended in ethylene diamine (50mls) and water (5mls). The reactants were stirred and refluxed for a total period of 12 hours. The mixture was allowed to cool and then diluted with 3 volumes of water, filtered and well washed with water, dilute hydrochloric acid, dilute sodium hydroxide and water until the pH of the washings reached 7.5. The solid was allowed to dry, whereupon the polymer beads shrank to give 15.6g dry reactive polymer beads.

Infra-red analysis showed the polymer to contain amide, nitrile and primary amine groups. Acid titration of the polymer showed 69 per cent conversion of nitrile to amide.

1 (c) Reaction of Amino-Containing Polymer with Glutaraldehyde

The polymer prepared above (8.6g) was suspended in water (8.3mls) at room temperature and 50 per cent glutaraldehyde solution was added (8.3mls). The mixture was stirred under reflux at room temperature for 2 hours.

The solid was filtered off and well washed with water. The polymer spheres need not be dried out before reaction with biologically active material.

1 (d) Reaction of Polymer Beads with Alkaline Phosphatase

A sample of the polymer beads prepared as in Example 1 (c) was mixed with an aqueous solution of the enzyme alkaline phosphatase. The mixture was stirred for about 2 hours, filtered, and the remaining beads washed with water.

The enzymatic activity of the immobilised alkaline phosphate enzyme was determined by measuring the amount of p-nitrophenol produced by the action of the immobilised enzyme on p-nitrophenyl phosphate in an aqueous solution having a pH value of 10.3. The absorbance of the solution was measured using a spectrophotometer and the amount of p-nitrophenol produced was calculated from the absorbance of the solution at 410mm. The activity was found to be 24 Units per gram of the enzyme-carrying polymer beads. 1 Unit is defined as 1 micromole of p-nitrophenol produced per minute at 32°C.

1 (e) Reaction of Polymer Beads with Lactase

Experiment 1 (d) was repeated with the exception that the alkaline phosphate enzyme was replaced by the enzyme

lactase.

The enzymatic activity of the immobilised lactase was determined by measuring the amount of o-nitrophenol produced by the action of the immobilised enzyme on o-nitrophenyl-β D-galactoside in an aqueous solution having a pH value of 4.5. The amount of o-nitrophenol produced was calculated from the absorbance of the solution at 520mm. The activity was found to be 450 Units per gram of the enzyme-carrying polymer beads. 1 Unit is defined as 1 micromole of o-nitrophenol produced per minute at 30°C.

Example 2

2 (a) Reaction of Nitrile-Containing Polymer with Ethanolamine

A suspension of polymer prepared as in Example 1(a) (3.0g), ethanolamine (40mls) and water (2mls) was stirred at 130°C for 7 hours. The mixture was allowed to cool, filtered, washed with water, dilute acid and water, then dried to give 3.6g of essentially spherical particles.

2 (b) Reaction of Hydroxy-Containing Polymer with Cyanogen Bromide

A portion of the polymer prepared as above (0.5g) was suspended in water (20mls), stirred and cooled to 3°C. A portion of 1N sodium hydroxide was added to give a pH of 10. Cyanogen bromide (0.4g) was added portionwise over 2 minutes. The mixture was stirred for a further 15 minutes. Portions of 1N sodium hydroxide solution were periodically added to maintain pH at 10. The material was filtered and washed with 0.1 N potassium bicarbonate solution.

## Example 3

### Reaction of Nitrile-Containing Polymer with Diethylene Triamine

A suspension of polymer prepared as in Example 1 (a) (10g), diethylene triamine (16g) and water (2mls) was stirred at 110°C for 12 hours. The resulting solid was filtered off and washed with water, dilute hydrochloric acid and dilute sodium hydroxide until the washings had a pH value of about 8-9.

Infra-red analysis showed the product to contain both amide and nitrile groups.

## Example 4

### 4 (a) Preparation of Nitrile-Containing Polymer Beads Using Acrylonitrile and N,N-Methylene Bis(acrylamide) Monomers

A mixture of distilled water (250mls), calcium carbonate (2.5g), sodium sulphate (15g), a 2 per cent gelatin solution (10mls), toluene (40mls), acrylonitrile (42.2g) and N,N-methylene bis (acrylamide) (6.9g) was stirred at room temperature for approximately 10 minutes to ensure adequate dispersion of the monomers. A portion (0.6g) of benzoyl peroxide containing 25 per cent water was added. The mixture was warmed to 75°C, held close to that temperature for 3.5 hours, and then further warmed to 86°C and held for an hour. The mixture was then allowed to cool, was filtered and the product washed with water and acetone and then dried to give 42.2g of small polymer beads and 5.2g of an amorphous powder.

Infra-red analysis showed the polymer beads to contain both nitrile and amide groups. The beads were insoluble in dimethyl formamide indicating that the beads comprised a cross-linked polymer.

4 (b) Reaction of Polymer of 4 (a) with Ethylene Diamine

A sample (10g) of the polymer beads prepared as in Example 4(a) was suspended in ethylene diamine (92mls) and water (8mls). The mixture was stirred for 1 hour and then refluxed for 3 hours. The resulting product was filtered off, well washed with water and dried to give 22g polymer beads.

The beads were shown by infra-red spectroscopy to contain a large proportion of amide groups and some nitrile groups.

## CLAIMS

1. A process for the preparation of water-insoluble porous polymer beads, the polymer containing functional groups bonded to the polymer as pendent groups of the formula:

$$-\overset{O}{\overset{\|}{C}}-\overset{R}{\overset{|}{N}}-A-X$$

where A represents a divalent aliphatic linking group having 2 to 10 carbon atoms, X represents a reactive functional group and R represents a hydrogen atom, an alkyl group or a group of the formula -A-X, characterised in that an unsaturated nitrile monomer and a cross-linking monomer containing at least two polymerisable double bonds are copolymerised in an organic phase dispersed in water, the organic phase comprising an inert, substantially water-immiscible organic liquid in which the monomers are soluble, to form water-insoluble porous polymer beads, and the polymer beads so formed are reacted with an amine of the formula:

$$H-\overset{R}{\overset{|}{N}}-A-X$$

where A, X and R are defined as above, in the presence of water to convert at least some of the nitrile groups of the polymer to groups of the formula:

$$-\overset{O}{\overset{\|}{C}}-\overset{R}{\overset{|}{N}}-A-X \quad .$$

2. A process according to claim 1, wherein the amount of unsaturated nitrile monomer present in the reaction mixture is at least 50 mole per cent based on the total amount of monomers present.

3. A process according to claim 1 or 2, wherein the unsaturated nitrile monomer is acrylonitrile or methacrylonitrile.

4. A process according to claim 1, 2 or 3, wherein the cross-linking monomer is substantially hydrophobic, and preferably is divinyl benzene.

5. A process according to any one of the preceding claims, wherein X represents an amine or hydroxyl group, preferably a primary amine group.

6. A process according to any one of the preceding claims, wherein the polymer beads are reacted with the amine in the presence of water to an extent such that 25 to 100 per cent of the nitrile groups in the polymer are converted to the groups of the formula:

$$-\overset{O}{\overset{\|}{C}}-\overset{R}{\overset{|}{N}}-A-X \quad .$$

7. Water-insoluble porous polymer beads comprising a cross-linked polymer having both nitrile pendent groups and substituted amide pendent groups of the formula:

$$-\overset{O}{\overset{\|}{C}}-\overset{R}{\overset{|}{N}}-A-X$$

where A represents a divalent aliphatic linking group having 2 to 10 carbon atoms, X represents a reactive functional group, preferably an amine or hydroxyl group, and R represents a hydrogen atom, an alkyl group or a group of the formula -A-X.

8. A process for the preparation of water-insoluble porous polymer beads, the polymer containing pendent groups containing a reactive group capable of bonding to a biologically active material, characterised in that water-insoluble porous polymer beads according to claim 7 or prepared by a process according to any of claims 1 to 6 are further reacted with a reagent, preferably an aldehyde, capable of bonding to a biologically active material.

9. Water-insoluble porous polymer beads comprising a cross-linked polymer having both nitrile pendent groups and pendent groups containing a reactive group capable of bonding to a biologically active material.

10. Polymer beads according to claim 9, wherein the reactive group is an aldehyde group.

11. The use of water-insoluble polymer beads according to claim 9 or 10 or prepared by a process according to claim 8 for the immobilization of an enzyme or other biologically active material.